# EUROPÄISCHE PATENTSCHRIFT

(11) **EP 1 069 142 B1**
(45) Veröffentlichungstag und Bekanntmachung des Hinweises auf die Patenterteilung: **28.04.2010**
(21) Anmeldenummer: 00114625.7
(22) Anmeldetag: 07.07.2000
(51) Int. Cl.: C08F 290/06, A61K 47/32, A61K 8/00, A01N 25/10

(54) **Wasserlösliche Polymere und ihre Verwendung in kosmetischen und pharmazeutischen Mitteln**
Water soluble polymers and their use in cosmetic and pharmaceutic products
Polymères solubles dans l'eau et leur utilisation dans des produits cosmétiques et pharmaceutiques

(30) Priorität: 15.07.1999 DE 19933066; 21.06.2000 DE 10029462
(43) Veröffentlichungstag der Anmeldung: 17.01.2001
(73) Patentinhaber: Clariant Produkte (Deutschland) GmbH, 65929 Frankfurt am Main (DE)
(72) Erfinder: Morschhäuser, Roman, Dr., 55122 Mainz (DE); Löffler, Matthias, Dr., 65527 Niedernhausen (DE)

(56) Entgegenhaltungen:
- EP-A- 0 850 894
- WO-A-94/24202
- WO-A-97/14448
- GB-A- 1 167 524
- US-A- 5 045 619
- US-A- 5 086 142
- US-A- 5 395 907
- US-A- 5 480 953
- US-A- 5 712 359
- US-A- 5 837 789
- US-A- 5 849 840

## Beschreibung

Die vorliegende Erfindung betrifft wasserlösliche Polymere, hergestellt durch Copolymerisation von Makromonomeren, und ihre Verwendung in kosmetischen und pharmazeutischen Mitteln.

In den letzten Jahren erlangten wasserlösliche Polymere eine immer größer werdende Bedeutung in Industrie und Wissenschaft. Polyelektrolyte nehmen dabei mengenmäßig einen sehr großen Teil der jährlichen Gesamtproduktion an wasserlöslichen Polymeren ein. Sie finden Verwendung als Flokkulantien in der Papierverarbeitung, der Waschmittelindustrie, der Textilverarbeitung, als Schutzkolloide oder in ihren vernetzten Varianten als Verdicker, um nur einige Anwendungsbereiche zu nennen.

Verdicker, insbesondere die auf der Basis der Polyacrylsäure hergestellten "Superabsorber" sind seit ihrer Entwicklung in den 70iger Jahren aus dem Hygienebereich nicht mehr wegzudenken. Neuere Verdicker, wie etwa die vernetzte Polyacrylamidopropylenmethylsulfonsäure (bzw. deren Salze), zeichnen sich durch ihre deutlich verbesserte pH-Stabilität und eine bessere Verarbeitbarkeit aus.

Zum Zwecke der Vereinfachung von kosmetischen Zubereitungen wird in den letzten Jahren verstärkt nach Rohstoffen gesucht, die mehrere Eigenschaften in einem Formulierungsbestandteil vereinen. Reine "Verdicker" werden durch neue Substanzen abgelöst, die in ihrem Eigenschaftsprofil beispielsweise noch Emulgatoreigenschaften aufweisen und damit einen Zusatz von separaten Emulgatoren in Formulierungen unnötig machen. Die Synthese der im folgenden beschriebenen Polymere bietet dazu das ideale Instrument. Die interessante Kombination eines Polyelektrolyten (z.B. Poly-AMPS) mit unpolaren Molekülteilen bietet die Möglichkeit einer Variation der hydrophil-hydrophoben Balance eines Polymeren, wie sie bisher in der synthetischen Polymerchemie nur in wenigen Fällen beobachtet wird.

Das Temperaturverhalten der Polymere ist eine wichtige Eigenschaft. Im allgemeinen zeigen Polymere bei niedrigen Temperaturen eine hohe Viskosität, und bei hohen Temperaturen eine niedrige Viskosität. Erwünscht sind oftmals aber solche Polymere, die oberhalb bestimmter Temperaturen verdickend wirken, aber bei niedrigen Temperaturen in Lösung pumpbar und verarbeitbar bleiben.

EP-A-0 583 814 und EP-A-0 629 649 offenbaren Polymere auf Basis von Acrylsäure als Hauptkette und Polyethylen- und/oder Polypropylenglykolen als Seitenketten. Diese Polymere zeigen eine Verdickung bei steigenden Temperaturen.

Alle Versuche zur Produktion solcher Polymere im industriellen Maßstab verliefen bisher erfolglos. Zudem war die Verwendung von acrylsäurehaltigen Hauptkettenpolymeren notwendig für die Realisierung des Konzeptes von thermoverdickenden Polymerlösungen in Wasser. Eines der gravierendsten Probleme bei dieser Polymerklasse sind Ausfällungserscheinungen aufgrund von Instabilitäten gegenüber zweiwertigen Ionen.

In der vorliegenden Erfindung werden nun eine neue Polymerklasse und ein Verfahren zu deren Herstellung beschrieben. Ohne großen präparativen Aufwand ist es mit diesem Verfahren auf Basis einer radikalischen Copolymerisation, insbesondere der Fällungspolymerisation, möglich, eine Vielzahl von neuen Polymeren mit unterschiedlichem thermischen Lösungsverhalten im industriellen Maßstab herzustellen.

Überraschenderweise wurde gefunden, dass Polymere, die den genannten Anforderungen genügen, auch mittels einer Makromonomersynthese darstellbar sind. Dies hat zur Folge, dass die nach dem Stand der Technik erforderliche Festlegung auf Acrylsäure als eines der Hauptkettenmonomere entfällt.

Gegenstand der Erfindung sind wasserlösliche Polymere, herstellbar durch radikalische Copolymerisation von
A) einem oder mehreren Makromonomeren der Formel (1)

   R¹-Y-(R²-O)ₓ-R³ (1)

   worin R¹ einen Acryl- oder Methacrylrest; R² (C₂-C₄)-Alkylen; x eine ganze Zahl zwischen 1 und 500; Y = O, S, PH oder NH; und R³ einen gesättigten oder ungesättigten linearen oder verzweigten aliphatischen oder cycloaliphatischen (C₆-C₃₀)-Kohlenwasserstoffrest bedeuten, und
B) einem oder mehreren olefinisch ungesättigten Comonomeren, die Sauerstoff, Stickstoff, Schwefel, Phosphor, Chlor und/oder Fluor enthalten.

US 5,837,789 offenbart superabsorbierende vernetzte Polymere für wässrige Flüssigkeiten, die aufgebaut sind aus teilweise neutralisierten Monomeren mit monoethylenisch ungesättigten sauren Gruppen, gegebenenfalls weiteren damit copolymerisierbaren Monomeren und Polymeren, die gegebenenfalls als Propfbasis geeignet sind. Gemäß der US 5,837,789 sind superabsorbierende Polymere wasserunlöslich.

EP 0 850 894 beschreibt als Zement-Additive Copolymere enthaltend ungesättigte Polyalkylenglykolether-basierende Monomere (I), Maleinsäure-basierende Monomere (II) und andere Monomere (III), die mit diesen Monomeren copolymerisierbar sind. In den Polyalkylenglykolether-basierenden Monomeren (I) ist die Polyalkylenglykol-Gruppierung über eine Etherbindung an den ungesättigten Molekülteil gebunden.

US 5,395,907 offenbart einen wasserlöslichen oder wasserdispergierbaren Haftkleber enthaltend ein Copolymer aus einem wasserlöslichen Basismonomer und einem wasserlöslichen Macromer. Das Macromer enthält Alkylenoxygruppen mit 2 bis 6 C-Atomen, an welche Wasserstoff -H oder C₁₋₅-Alkylgruppen gebunden sind. GB 1,167,524 beschreibt ein Verfahren zur Herstellung von wasser- oder alkalilöslichen oder quellbaren Copolymeren. Beispielhaft wird u. a. die Herstellung von Copolymeren aus Polyethylenglykol monomethacrylat monononylphenylether mit Maleinsäureanhydrid und Methylvinylether oder mit Styrol und Maleinsäureanhydrid oder mit Acrylamid offenbart.

R² steht besonders bevorzugt für einen Ethylen- oder Propylenrest. x steht besonders bevorzugt für eine Zahl zwischen 3 und 50, insbesondere bevorzugt für eine Zahl zwischen 7 und 30. Bei R³ handelt es sich um aliphatische oder cycloaliphatische Kohlenwasserstoffe, die gesättigt oder ungesättigt sein können.
R³ steht besonders bevorzugt für einen (C₆-C₂₂)-Kohlenwasserstoffrest, insbesondere bevorzugt für einen (C₁₂-C₁₈)-Kohlenwasserstoffrest.

Die Herstellung der Makromonomeren erfolgt im allgemeinen durch die Reaktion reaktiver Derivate der (Meth)Acrylsäure mit Hydroxylgruppen enthaltenden Verbindungen, insbesondere alkoxylierten Alkylresten. Auch die ringöffnende Addition an (Meth)Acrylsäureglycidylester ist möglich.

Als olefinisch ungesättigte Comonomere B) eignen sich bevorzugt olefinisch ungesättigte Säuren und deren Salze mit ein- und zweiwertigen Gegenionen, besonders bevorzugt Styrolsulfonsäure, Acrylamidopropylmethylensulfonsäure (AMPS), Vinylsulfonsäure, Vinylphosphonsäure, Allylsulfonsäure, Methallylsulfonsäure, Acrylsäure, (Meth)acrylsäure, Maleinsäure bzw. Maleinsäureanhydrid und deren Salze; Ester der Acryl- und der (Meth)acrylsäure mit aliphatischen, aromatischen oder cyclo-aliphatischen Alkoholen mit einer Kohlenstoffzahl von 1 bis 22; Ester der Acryl- und der (Meth)acrylsäure mit Alkylethoxylaten, offenkettige und cyclische N-Vinylamide (N-Vinyllactame) mit einer Ringgröße von 4 bis 9 Atomen, besonders bevorzugt N-Vinylformamid (NVF), N-Vinylmethylformamid, N-Vinyl-methylacetamid (VIMA), N-Vinylacetamid, N-Vinylpyrrolidon (NVP) und N-Vinylcaprolactam; Amide der Acryl- und der Methacrylsäure, besonders bevorzugt Acrylamid, N,N-Dimethylacrylamid, N,N-Diethylacrylamid, alkoxylierte Acryl- und Methacrylamide, wie z.B. MAPTAC und APTAC; 2-Vinylpyridin; 4-Vinylpyridin; Vinylacetat; Methacrylsäureglycidylester; Acrylnitril; Vinylchlorid; Vinylidenchlorid; Tetrafluorethylen und/oder DADMAC.

Als Gegenionen für die Salze der olefinisch ungesättigten Säuren eignen sich bevorzugt Lithium, Natrium, Kalium, Magnesium, Calcium, Ammonium, Monoalkylammonium, Dialkylammonium, Trialkylammonium oder Tetraalkylammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann. Ebenfalls geeignet sind ein bis dreifach ethoxylierte Ammoniumverbindungen mit unterschiedlichem Ethoxylierungsgrad. Als Gegenionen besonders bevorzugt sind Natrium und Ammonium. Der Neutralisationsgrad der olefinisch ungesättigten Säuren beträgt bevorzugt zwischen 70 und 100 Mol %.

Besonders bevorzugt sind wasserlösliche Polymere, herstellbar durch radikalische Copolymerisation von
A) einem oder mehreren Makromonomeren, ausgewählt aus der Gruppe der Ester der (Meth)acrylsäure mit Alkylethoxylaten, die 5 bis 80 EO-Einheiten und (C₁₀-C₂₂)-Alkylreste umfassen, und
B) einem oder mehreren olefinisch ungesättigten Comonomeren, ausgewählt aus der Gruppe Acrylamidopropylmethylensulfonsäure (AMPS), Natrium- und Ammoniumsalze der Acrylamidopropylmethylensulfonsäure (AMPS), Acrylamid, N-Vinylformamid, N-Vinylmethylacetamid und/oder Natriummethallylsulfonat.

Als Makromonomere A) eignen sich hierbei insbesondere Ester der (Meth)acrylsäure mit
(C₁₀-C₁₈)-Fettalkoholpolyglykolether mit 8 EO-Einheiten (Genapol^{®} C-080)
C₁₁-Oxoalkoholpolyglykolether mit 8 EO-Einheiten (Genapol^{®} UD-080)
(C₁₂-C₁₄)-Fettalkoholpolyglykolether mit 7 EO-Einheiten (Genapol^{®} LA-070)
(C₁₂-C₁₄)-Fettalkoholpolyglykolether mit 11 EO-Einheiten (Genapol^{®} LA-110)
(C₁₆-C₁₈)-Fettalkoholpolyglykolether mit 8 EO-Einheiten (Genapol^{®} T-080)
(C₁₆-C₁₈)-Fettalkoholpolyglykolether mit 15 EO -Einheiten (Genapol^{®} T-1 50)
(C₁₆-C₁₈)-Fettalkoholpolyglykolether mit 11 EO-Einheiten (Genapol^{®} T-110)
(C₁₆-C₁₈)-Fettalkoholpolyglycolether mit 20 EO-Einheiten (Genapol^{®} T-200)
(C₁₆-C₁₈)-Fettalkoholpolyglycolether mit 25 EO-Einheiten (Genapol^{®} T-250)
(C₁₈-C₂₂)-Fettalkoholpolyglycolether mit 25 EO-Einheiten
und/oder iso-(C₁₆-C₁₈)-Fettalkoholpolyglycolether mit 25 EO-Einheiten und als olefinisch ungesättigte Comonomere B) eignen sich insbesondere die Natrium- und Ammoniumsalze der Acrylamidopropylmethylensulfonsäure (AMPS). Bei den Genapol^{®}-Typen handelt es sich um Produkte der Firma Clariant.

Die molaren Anteile der Makromonomeren A) und der Comonomeren B) im Polymer kann zwischen 0,1 und 99,9 mol-% variieren.
In einer bevorzugten Ausführungsform (hoch hydrophob modifizierte Polymere) beträgt der Anteil an Makromonomeren A) 50,1 bis 99,9 mol-%, bevorzugt 70 bis 95 mol-%, besonders bevorzugt 80 bis 90 mol-%.
In einer weiteren bevorzugten Ausführungsform (niedrig hydrophob modifizierte Polymere) beträgt der Anteil an Makromonomeren A) 0,1 bis 50 mol-%, bevorzugt 5 bis 25 mol-%, besonders bevorzugt 10 bis 20 mol-%.

Die Monomerenverteilung der Makromonomeren A) und Comonomeren B) in den Polymeren kann beispielsweise alternierend, blockartig (auch Multiblock) oder auch statistisch (auch Gradient) sein.
Die Polymere weisen im allgemeinen ein zahlenmittleres Molekulargewicht von 1000 bis 20.000.000 g/mol, bevorzugt 20.000 bis 5.000.000, insbesondere bevorzugt 100.000 bis 1.500.000 g/mol, auf.

In einer bevorzugten Ausführungsform sind die erfindungsgemäßen Polymere vernetzt, d.h. sie enthalten mindestens einen Vernetzer mit mindestens zwei Doppelbindungen, der in das Polymer einpolymerisiert ist. Geeignete Vernetzer sind insbesondere Methylenbisacryl- und -methacrylamid, Ester ungesättigter Mono- oder Polycarbonsäuren mit Polyolen, z.B. Diacrylate oder Triacrylate, wie z.B. Butandiol- und Ethylenglykoldiacrylat bzw. -methacrylat und Trimethylolpropantriacrylat, Allylverbindungen, wie z.B. Allyl(meth)acrylat, Triallylcyanurat, Maleinsäurediallylester, Polyallylester, Tetraallyloxyethan, Triallylamin, Tetraallylethylendiamin, Allylester der Phosphorsäure und/oder Vinylphosphonsäurederivate.

Eine bevorzugte Ausführungsform der Erfindung stellen solche Polymere dar, die aus einem wasserlöslichen Polymerskelett und Makromonomeren A) mit wärmeempfindlichen Seitenketten, die in Wasser ein LCST-Verhalten aufweisen, bestehen, und deren wässrige Lösung eine Viskosität hat, die oberhalb einer bestimmten Schwellentemperatur mit steigender Temperatur zunimmt oder ungefähr konstant bleibt.

Eine weitere bevorzugte Ausführungsform der Erfindung stellen solche Polymere dar, deren wässriger Lösung eine Viskosität hat, die unterhalb einer ersten Schwellentemperatur niedrig ist, oberhalb dieser ersten Schwellentemperatur mit steigender Temperatur zu einem Maximum ansteigt und oberhalb einer zweiten Schwellentemperatur mit steigender Temperatur wieder abfällt. Dabei ist bevorzugt, dass die Viskosität der Polymerlösungen unterhalb der ersten Schwellentemperatur bei 5 bis 50 %, insbesondere 10 bis 30 %, der Maximalviskosität bei der zweiten Schwellentemperatur liegt.

Ebenfalls eine bevorzugte Ausführungsform der Erfindung stellen solche Polymere dar, deren wässrige Lösungen bereits bei Raumtemperatur eine hohe Viskosität und kein thermoassoziierendes Verhalten aufweisen.
Bevorzugt betragen die Viskositäten der 1% igen wässrigen Lösungen 20 000 mPas bis 100 000 mPas, insbesondere 60 000 mPas bis 70 000 mPas.
Solche Polymere zeigen bereits bei Raumtemperatur eine hohe Verdickerleistung, gute Emulgiereigenschaften und gute Dispersionseigenschaften in wässriger, wässrig-alkoholischer und wässrig-tensidischer Lösung oder in Emulsionen.
Des weiteren zeigen Zubereitungen, enthaltend solche Polymere, eine gute Transparenz und eine hohe Elektrolytstabilität.

Die erfindungsgemäßen Polymere werden durch radikalische Copolymerisation, wie z.B. Fällungspolymerisation, Emulsionspolymerisation, Lösungspolymerisation oder Suspensionspolymerisation hergestellt. Bevorzugt eignet sich die Fällungspolymerisation, besonders bevorzugt die Fällungspolymerisation in tert.-Butanol.

Mit Hilfe der Fällungspolymerisation in tert.-Butanol lässt sich eine für die Verwendung der Polymere besonders günstige Partikelgrößenverteilung der Polymere erreichen. Die Größenverteilung der Polymerpartikel kann z.B. durch Laserebeugung oder Siebanalyse bestimmt werden. Repräsentativ für eine günstige Größenverteilung ist die folgende Korngrößenverteilung, wie sie durch Siebanalyse für ein AMPS-Copolymer bestimmt wurde: 60,2 % kleiner 423 Mikrometer, 52,0 % kleiner 212 Mikrometer, 26,6 % kleiner 106 Mikrometer, 2,6 % kleiner 45 Mikrometer und 26,6 % größer 850 Mikrometer.

Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert.-Butylhydroperoxid, Methylethylketonperoxid, Cumolhydroperoxid, Azoverbindungen, wie z.B. Azobisisobutyronitril, Azobisdimethylvaleronitril, sowie anorganische Peroxiverbindungen, wie z.B. (NH₄)₂S₂O₈, K₂S₂O₈ oder H₂O₂, gegebenenfalls in Kombination mit Reduktionsmitteln, wie z.B. Natriumhydrogensulfit und Eisen(II)-sulfat, oder Redoxsystemen, welche als reduzierende Komponente eine aliphatische oder aromatische Sulfonsäure, wie z.B. Benzolsulfonsäure, Toluolsulfonsäure oder Derivate dieser Säuren, wie z.B. Mannichaddukte aus Sulfinsäure, Aldehyden und Aminoverbindungen, enthalten.

Die erfindungsgemäßen Polymere eignen sich als Verdicker und Dispergiermittel für wässrige Zubereitungen, wässrig-alkoholische und wässrige-tensidische Zubereitungen und als Emulgatoren, Suspendiermittel mit verdickender Wirkung und Konsistenzgeber für Emulsionen und Suspensionen. Dabei können auch Mischungen der Polymere verwendet werden. Insbesondere eignen sich die Polymere zur Verwendung in kosmetischen und pharmazeutischen Mitteln.
Wichtig ist dabei, dass die Polymere ohne Mitverwendung eines zusätzlichen Co-Emulgators und/oder ohne Mitverwendung eines zusätzlichen Konsistenzgebers eingesetzt werden können. Die Mitverwendung von Co-Emulgatoren und Konsistenzgebem ist daher nicht zwingend, aber möglich.

Gegenstand der Erfindung sind somit auch wässrige Zubereitungen, wässrig-alkoholische Zubereitungen, wässrig-tensidische Zubereitungen, Emulsionen und Suspensionen, enthaltend die erfindungsgemäßen Polymere.
Bei den Zubereitungen, Emulsionen und Suspensionen handelt es sich bevorzugt um kosmetische und pharmazeutische Mittel, wie z.B. Shampoos, Duschbäder, Duschgels, Schaumbäder, Gels, Lotions, Cremes und Salben.

Die erfindungsgemäßen Zubereitungen, Emulsionen und Suspensionen enthalten, bezogen auf die fertige Formulierung, bevorzugt 0,05 bis 10 Gew.-%, besonders bevorzugt 0,1 bis 5 Gew.-%, insbesondere bevorzugt 0,5 bis 3 Gew.-%, an erfindungsgemäßen Polymeren.

Die erfindungsgemäßen Polymere können als Verdicker für Mittel auf wässriger oder wässrig-alkoholischer Basis, beispielsweise Haargele, eingesetzt werden. Des weiteren eignen sich die erfindungsgemäßen Polymere als Verdicker, Dispergiermittel und Konsistenzgeber für wässrig-tensidische Zubereitungen, beispielsweise Shampoos, Duschbäder, Duschgels, Schaumbäder und dergleichen. Die verdickende Wirkung der erfindungsgemäßen Polymere in wässrig-tensidischen Mitteln wird durch eine Assoziation der Polymerseitenketten und der Tenside verstärkt und kann durch die Wahl der Seitenketten der Polymere und durch die Wahl der Tenside gesteuert werden. Die suspendierende bzw. dispergierende und stabilisierende Wirkung der erfindungsgemäßen Polymere in wässrig-tensidischen Mitteln wird durch die Assoziation der Polymerseitenketten und der in wässrig-tensidischen Mitteln unlöslichen flüssigen Komponenten, beispielsweise Silikonöle, bzw. der unlöslichen Komponenten, beispielsweise Zink-Pyrethione, bedingt.

Die erfindungsgemäßen Mittel können als weitere Hilfs- und Zusatzstoffe alle üblichen anionischen, kationischen, zwitterionischen, nicht-ionischen und amphotären Tenside, sowie weitere in der Kosmetik gebräuchliche Zusätze, wie z.B. Überfettungsmittel, Stabilisatoren, biogene Wirkstoffe, Glycerin, Konservierungsmittel, Perlglanzmittel, Farb- und Duftstoffe, Lösungsmittel, Trübungsmittel, weitere Verdickungsmittel und Dispergiermittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden

Die Gesamtmenge der in den erfindungsgemäßen Mitteln eingesetzten Tenside kann, bezogen auf das fertige Mittel, zwischen 5 und 70 Gew.-%, bevorzugt zwischen 10 und 40 Gew.-%, besonders bevorzugt zwischen 12 und 35 Gew.-%, betragen.
Als anionische waschaktive Substanzen seien genannt: (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside in den erfindungsgemäßen Mitteln liegt bevorzugt im Bereich von 7 bis 30 Gew.-%, besonders bevorzugt 10 bis 25 Gew.-%, insbesondere bevorzugt 12 bis 22 Gew.-%.

Geeignete kationische Tenside sind beispielsweise quartäre Ammonium-Salze wie Di-(C₁₀-C₂₄)-Alkyl-dimethylammoniumchlorid oder -bromid, vorzugsweise Di-(C₁₂-C₁₈)-Alkyl-dimethylammoniumchlorid oder -bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyl-dimethylbenzylammoniumchlorid oder -bromid, vorzugsweise (C₁₂-C₁₈)-Alkyl-dimethylbenzylammoniumchlorid; N-(C₁₀-C₁₈)-Alkyl-pyridiniumchlorid oder -bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyolaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside in den erfindungsgemäßen Mitteln liegt bevorzugt im Bereich von 1 bis 10 Gew.-%, besonders bevorzugt 2 bis 7 Gew.-%, insbesondere besonders bevorzugt 3 bis 5 Gew.-%.

Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkylolamide (Fettsäureamidpolyethylenglykole); N-Alkyl-, N-Alkoxypolyhydroxyfettsäureamide, Saccharoseester; Sorbitester und Polyglykolether.

Der Gewichtsanteil der nichtionischen Tenside in den erfindungsgemäßen Mitteln liegt bevorzugt im Bereich von 1 bis 20 Gew.-%, besonders bevorzugt 2 bis 10 Gew.-%, insbesondere bevorzugt 3 bis 7 Gew.-%.

Bevorzugte Amphotenside sind: N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol^{®}, Steinapon^{®}), vorzugsweise das Natrium-Salz des 1 -(β-Carboxy-methyloxyethyl)-1 - (carboxymethyl)-2-lauryl-imidazoliniums; Aminoxide, z.B. (C₁₂-C₁₈)-Alkyldimethylaminoxid, Fettsäureamidoalkyl-dimethylaminoxid.

Der Gewichtsanteil der amphotären Tenside in den erfindungsgemäßen Mitteln liegt bevorzugt im Bereich von 0,5 bis 20 Gew.-%, besonders bevorzugt 1 bis 10 Gew.-%.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind Laurylsulfat, Laurethsulfat, Cocoamidopropylbetain, Natriumcocoylglutamat, Di-natriumlaurethsulfosuccinat und Cocosfettsäurediethanolamid.
Die erfindungsgemäßen Zubereitungen können außerdem weitere in der Kosmetik gebräuchliche Zusätze wie Überfettungsmittel, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.
Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure.
Als Perlglanzmittel kommen beispielsweise Glycoldistearinsäureester, wie Ethylenglycoldistearat, aber auch Fettsäuremonoglycolester in Betracht.
Als Farbstoffe können die für kosmetische Zwecke geeigneten und zugelassenen Substanzen verwendet werden.

Als weitere Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol 934, -940, -941, -956, -980, -981, -1342, -1382, Ethylenglycolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat,
N,N-Dihydrocarbyl (C₁₂-C₂₂)- insbesondere (C₁₆-C₁₈)--amidobenzoesäure und deren lösliche Salze, N,N-di(C₁₆-C₁₈)-amidobenzoesäure und deren Derivate.
Die Dispergiermittel werden, bezogen auf das fertige Mittel, in Konzentrationen von bevorzugt 0,5 bis 10 Gew.-%, besonders bevorzugt von 0,5 bis 5 Gew.-%, insbesondere bevorzugt von 1 bis 4 Gew.-%, eingesetzt.
Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol.

Als Trägermaterialien in Betracht kommen pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und CelluloseDerivate.

Als fungizide Wirkstoffe können Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine eingesetzt werden.

Silicone, Polyalkylsiloxane, Polyalkylarylsiloxane, Polyethersiloxan-Copolymere, wie in US 5 104 645 und den darin zitierten Schriften beschrieben, verbessern noch die pflegende Wirkung der erfindungsgemäßen Mittel. Die erfindungsgemäßen Mittel können mit konventionellen Ceramiden, Pseudoceramiden, Fettsäure-N-alkylpolyhydroxyalkylamide, Cholesterin, Cholesterinfettsäureestern, Fettsäuren, Triglyceriden, Cerebrosiden, Phospholipiden und ähnlichen Stoffen, abgemischt werden.

Die erfindungsgemäßen Polymere können als Emulgatoren, Stabilisatoren und/oder Konsistenzgeber in Emulsionen eingesetzt werden. Insbesondere sind dafür vernetzte Polymere geeignet. Bei den Emulsionen kann es sich sowohl um Wasserin-Öl-Emulsionen als auch Öl-in-Wasser-Emulsionen handeln.
Die emulgierende, stabilisierende und/ oder konsistenzgebende Wirkung der erfindungsgemäßen Polymere in Emulsionen wird durch eine Assoziation der Polymerseitenketten untereinander, sowie durch eine Wechselwirkung der Polymerseitenketten mit den hydrophoben Ölkomponenten verursacht bzw. verstärkt.

Der nichtwässrige Anteil der Emulsionen, der sich weitgehend aus dem Emulgator, dem Verdicker und dem Ölkörper zusammensetzt, liegt üblicherweise bei 5 bis 95 %, vorzugsweise bei 15 bis 75 Gew.-%. Daraus folgt, dass die Emulsionen 5 bis 95 Gew.-% und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Ölkörper, Co-Emulgatoren, Überfettungsmittel, Fette, Wachse, Stabilisatoren, biogene Wirkstoffe, Glycerin, Konservierungsmittel, Farb- und Duftstoffe enthalten.

Als Ölkörper kommen beispielsweise Guerbetalkohole auf Basis von Fettalkoholen mit 6 bis 18, vorzugsweise 8 bis 10 Kohlenstoffatomen, Ester von linearen (C₆-C₁₃)-Fettsäuren mit linearen (C₆-C₂₀) Fettalkoholen, Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis (C₆-C₁₀)-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht. Der Anteil der Ölkörper am nichtwässrigen Anteil der Emulsionen kann 5 bis 95 und vorzugsweise 15 bis 75 Gew.-% ausmachen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.
Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.
Die Herstellung der Emulsionen kann in bekannter Weise, d.h. beispielsweise durch Heiß-, Heiß/Kalt- bzw. PIT-Emulgierung erfolgen.

Die erfindungsgemäßen Polymere können auch für die Formulierung von Pflanzenschutzmitteln verwendet werden. In diesem Anwendungsbereich kam es in den letzten Jahren zu einem Umdenken bei der Entwicklung neuer Wirkstoffformulierungen. Nicht die Forschung nach neuen Wirkstoffen stand im Vordergrund, sondern die Suche nach Hilfsreagenzien zur Wirkungsverstärkung von bekannten Wirkstoffen an der Pflanzenoberfläche. Der Zusatz dieser sogenannten Adjuvantien ermöglicht die Reduktion der eingesetzten Wirkstoffmenge unter Beibehaltung der Wirksamkeit der Gesamtformulierung im Vergleich zu Adjuvantienfreien Formulierungen. Unpolar modifizierte wasserlösliche Polyelektrolyte ermöglichen einerseits eine Viskositätserhöhung der vorliegenden Wirkstofflösung - was zu einem langsameren "Abperlen" von den Blattoberflächen führt und damit zu einer verlängerten Einwirkzeit auf das Blatt - und andererseits bewirken die unpolaren Seitenreste eine erhöhte Adsorption an die ebenfalls unpolaren Blattoberflächen.

Die nachfolgenden Beispiele und Anwendungen sollen die Erfindung näher erläutern, ohne sie jedoch darauf zu beschränken.

### Beispiele und Anwendungen

### 1) Darstellung der Makromonomeren

### a) Variante 1: Methacrylsäureglyzidylester

In einem ein Liter Dreihalskolben mit Rührer, Innenthermometer und Rückflusskühler werden 600 g Genapol T-250 vorgelegt und mit 75 g Methacrylsäureglyzidylester versetzt. Anschließend wird die Reaktionsmischung 2 Stunden lang auf 100°C erhitzt und der überschüssige Methacrylsäureglyzidylester im Vakuum abdestilliert. Das entstandene Makromonomere kann ohne weitere Reinigung in die Polymerisation eingesetzt werden.

### b) Variante 2: freie Meth-/Acrylsäure

In einem ein Liter Dreihalskolben mit Rührer, Innenthermometer und Rückflusskühler werden 500 g Genapol UD-070 vorgelegt und mit 100 g Meth-/Acrylsäure und p-Toluolsulfonsäure als Katalysator versetzt. Anschließend wird die Reaktionsmischung 2 Stunden unter Rückfluss gekocht und die überschüssige Säure und das entstehende Reaktionswasser im Vakuum abdestilliert. Das entstandene Makromonomere kann ohne weitere Reinigung in die Polymerisation eingesetzt werden.

### c) Variante 3: Halogenderivate der Meth-/Acrylsäure

In einem ein Liter Dreihalskolben mit Rührer, Innenthermometer und Rückflusskühler werden 500 g Genapol UD-070 mit einer primären Aminoendgruppe vorgelegt und mit 110 g Meth-/Acrylsäurechlorid und 50g Natriumcarbonat versetzt. Anschließend wird die Reaktionsmischung 2 Stunden unter Rückfluss gekocht. Das Aufhören der CO₂-Entwicklung indiziert das Ende der Modifizierungsreaktion. Das überschüssige Säurechlorid wird im Vakuum abdestilliert. Das entstandene Makromonomere mit einer Meth-/Acrylamidendguppe kann ohne weitere Reinigung in die Polymerisation eingesetzt werden.

### d) Variante 4: Ester der Meth-/Acrylsäure

In einem ein Liter Dreihalskolben mit Rührer, Innenthermometer und Rückflusskühler werden 500 g Genapol LA-090 vorgelegt und mit 100 g Meth-/Acrylsäuremethylester und 20 g Titantetraisopropylat versetzt. Anschließend wird die Reaktionsmischung 2 Stunden unter Rückfluss gekocht. Nachdem der entstehende Alkohol abdestilliert wurde wird der verbliebene Ester im Vakuum abdestilliert. Das entstandene Makromonomere mit einer Meth-/Acrylsäureendguppe kann ohne weitere Reinigung in die Polymerisation eingesetzt werden.

### 2) Polymerisation

Allgemeine Polymerisationsvorschrift zur Herstellung der erfindungsgemäßen Seitenkettenpolymere nach dem Fällungsverfahren in t-Butanol

In einem 2-Liter Quickfitkolben mit Rückflusskühler, Gaseinleitung, Innenthermometer und Rührer werden 500 ml t-Butanol vorgelegt und mit der berechneten Menge an AMPS versetzt. Anschließend wird durch NH₃-Einleitung neutralisiert und die wie unter 1) hergestellten LCST-Seitenarme (auch mehrere unterschiedliche Spezies möglich) in die Reaktionsmischung hinzugegeben. Sollten weitere Comonomere benötigt werden, dann können diese nach der Neutralisation in die Reaktionsmischung hinzugegeben werden. Nach Inertisierung der Mischung mit N₂ oder Argon wird bei einer Innentemperatur von 60°C AIBN als Initiator zugesetzt und die Polymerisationsreaktion eingeleitet. Nach wenigen Minuten kommt es zum Ausfällen des fertigen Polymeren. Die Mischung wird zwei Stunden auf Rückfluss erhitzt und das Polymerisat anschließend über eine Nutsche vom Lösungsmittel befreit und im Vakuum getrocknet. Diese Vorschrift ist allgemein auf alle im weiteren beschriebenen Polymerisationsreaktionen anwendbar.

### Seitenkettenpolymere mit verdickenden Eigenschaften

### Beispiel 1: Reaktion gemäß allgemeiner Polymerisationsvorschrift

| Reaktand | Menge (g) |
|---|---|
| Makromonomer Variante 1 - Typ Genapol^{®}T-250 | 20 |
| NH₃-neutralisiertes AMPS | 100 |
| t-Butanol | 300 |
| AIBN (Initiator) | 1 |

### Beispiel 2: Reaktion gemäß allgemeiner Polymerisationsvorschrift

| Reaktand | Menge (g) |
|---|---|
| Makromonomer Variante 3 - Typ Genapol^{®} UD-80 | 30 |
| NH₃-neutralisiertes AMPS | 90 |
| t-Butanol | 300 |
| AIBN (Initiator) | 1 |

### Beispiel 3: Reaktion gemäß allgemeiner Polymerisationsvorschrift

| Reaktand | Menge (g) |
|---|---|
| Makromonomer Variante 3 - Typ Genapol^{®} T-250 | 30 |
| NH₃-neutralisiertes AMPS | 90 |
| Methylenbisacrylamid (Vernetzer) | 1.5 |
| t-Butanol | 300 |
| AIBN (Initiator) | 1 |

### Beispiel 4: Reaktion gemäß allgemeiner Polymerisationsvorschrift

| Reaktand | Menge (g) |
|---|---|
| Makromonomer Variante 1 - Typ Genapol^{®}L-070 | 20 |
| Na-neutralisiertes AMPS | 75 |
| Acrylamid | 50 |
| t-Butanol | 300 |
| AIBN (Initiator) | 1 |

### Beispiel 5: Reaktion gemäß allgemeiner Polymerisationsvorschrift

| Reaktand | Menge (g) |
|---|---|
| Makromonomer Variante 4 - Typ Genapol^{®}UD-080 | 15 |
| Makromonomer Variante 1 - Typ Genapol^{®}T-250 | 15 |
| NH₃-neutralisiertes AMPS | 100 |
| t-Butanol | 300 |
| AIBN (Initiator) | 1 |

### Beispiel 6: Reaktion gemäß allgemeiner Polymerisationsvorschrift

| Reaktand | Menge (g) |
|---|---|
| Makromonomer Variante 1 - Typ Genapol^{®}LA-110 | 20 |
| NH₃-neutralisiertes AMPS | 100 |
| t-Butanol | 300 |
| AIBN (Initiator) | 1 |

### Beispiel 7: Reaktion gemäß allgemeiner Polymerisationsvorschrift

| Reaktand | Menge (g) |
|---|---|
| Makromonomer Variante 1 - Typ Genapol^{®}T-080 | 20 |
| NH₃-neutralisiertes AMPS | 100 |
| t-Butanol | 300 |
| AIBN (Initiator) | 1 |

### Beispiel 8: Reaktion gemäß allgemeiner Polymerisationsvorschrift

| Reaktand | Menge (g) |
|---|---|
| Makromonomer Variante 1 - Typ Genapol^{®}T-150 | 25 |
| NH₃-neutralisiertes AMPS | 100 |
| AM | 10 |
| t-Butanol | 350 |
| AIBN (Initiator) | 1 |

### Beispiel 9: Reaktion gemäß allgemeiner Polymerisationsvorschrift

| | 9,1 | 9,2 | 9,3 |
|---|---|---|---|
| Reaktand | Menge (g) | Menge (g) | Menge (g) |
| Makromonomer Variante 1 - Typ Genapol^{®}LA-110 | 10 | 20 | 10 |
| Makromonomer Variante 1 - Typ Genapol^{®}T-150 | 12,5 | 12,5 | 25 |
| NH₃-neutralisiertes AMPS | 100 | 100 | 100 |
| AM | 10 | 10 | 10 |
| t-Butanol | 350 | 350 | 350 |
| AIBN (Initiator) | 1 | 1 | 1 |

### Beispiel 10: Polymermischung

| | 10.1 | 10.2 | 10.3 |
|---|---|---|---|
| Polymer | Menge (g) | Menge (g) | Menge (g) |
| Beispiel 1 | 10 | 20 | 10 |
| Beispiel 3 | 10 | 10 | 20 |

### Beispiel 11: Reaktion gemäß allgemeiner Polymerisationsvorschrift jedoch bei 45°C Starttemperatur

| Reaktand | Menge (g) |
|---|---|
| Makromonomer Variante 1 - Typ (C₁₈-C₂₂)-Fettalkoholpolyglycolether mit 25 EO-Einheiten | 25 |
| NH₃-neutralisiertes AMPS | 100 |
| t-Butanol | 300 |
| Azobisdimethylvaleronitril (Initiator) | 1 |

### Beispiel 12: Reaktion gemäß allgemeiner Polymerisationsvorschrift jedoch bei 45°C Starttemperatur

| Reaktand | Menge (g) |
|---|---|
| Makromonomer Variante 1 - Typ Genapol^{®}UD-080 | 20 |
| NH₃-neutralisiertes AMPS | 100 |
| t-Butanol | 300 |
| Azobisdimethylvaleronitril (Initiator) | 1 |

### Beispiel 13: Reaktion gemäß allgemeiner Polymerisationsvorschrift

| Reaktand | Menge (g) |
|---|---|
| Makromonomer Variante 1 - Typ Genapol^{®}T-200 | 15 |
| NH₃-neutralisiertes AMPS | 100 |
| t-Butanol | 300 |
| AIBN (Initiator) | 1 |

### Beispiel 14: Reaktion gemäß allgemeiner Polymerisationsvorschrift

| Reaktand | Menge (g) |
|---|---|
| Genapol^{®}T-250-methacrylat | 97 |
| NH₃-neutralisiertes AMPS | 3 |
| t-Butanol | 300 |
| TMPTA | 1,8 |
| Dilauroylperoxid (Initiator) | 1 |

### Beispiel 15 (Vergleich): Reaktion gemäß allgemeiner Polymerisationsvorschrift

| Reaktand | Menge (g) |
|---|---|
| MPEG 750-Methacrylat | 80 |
| NH₃-neutralisiertes AMPS | 20 |
| t-Butanol | 300 |
| TMPTA | 1.8 |
| AIBN (Initiator) | 1 |

| | |
|---|---|
| Viskosität 1,0 %ig (H₂O dest.) 55.000 mPas (Brookfield Spindel 7) Viskosität 0,5%ig (H₂O dest.) 19.000 mPas (Brookfield Spindel 7) | |

### Beispiel 16: Reaktion gemäß allgemeiner Polymerisationsvorschrift

| Reaktand | Menge (g) |
|---|---|
| Genapol^{®}T-250-methacrylat | 97 |
| NH₃-neutralisiertes AMPS | 3 |
| t-Butanol | 300 |
| Dilauroylperoxid (Initiator) | 1 |

### Beispiel 17 (Vergleich): Reaktion gemäß allgemeiner Polymerisationsvorschrift

| Reaktand | Menge (g) |
|---|---|
| MPEG-750-methacrylat | 20 |
| NH₃-neutralisiertes AMPS | 80 |
| t-Butanol | 300 |
| ABAH (Initiator) | 1 |

| | |
|---|---|
| Viskosität 1,0%ig (H₂O dest.) 1.500 mPas (Brookfield Spindel 5) Viskosität 0,5%ig (H₂O dest.) 400 mPas (Brookfield Spindel 5) | |

### Vergleichsbeispiel 1:

Das Vergleichsbeispiel 1 zeigt, dass die Temperatur, bei der die Viskosifizierung einsetzt und die Stärke des Effekts beeinflusst werden kann (Abbildung 1). Die verwendeten Polymere basieren auf den Beispielen 6, 7 und 8. Zusätzlich wird ein Polymer aus EP-B-0 583 814 nachgestellt. Es handelt sich dabei um pAMPS/1,2-POE5 aus Beispiel 1.3. Abbildung 1 zeigt die Viskosität der Polymere in wässriger Lösung. In Abbildung 1 ist zu erkennen, dass die Maxima der Viskosifizierung bei unterschiedlichen Temperaturen zu finden sind: für Beispiel 6 bei 120°C, für Beispiel 7 bei 140°C und für Beispiel 8 bei über 200°C. pAMPS/1.2-POE5 weist sein Temperaturmaximum bei 200°C auf. Die Einsatzkonzentrationen sind darüber hinaus deutlich geringer als bei pAMPS/1.2-POE5 (Abbildung 1).

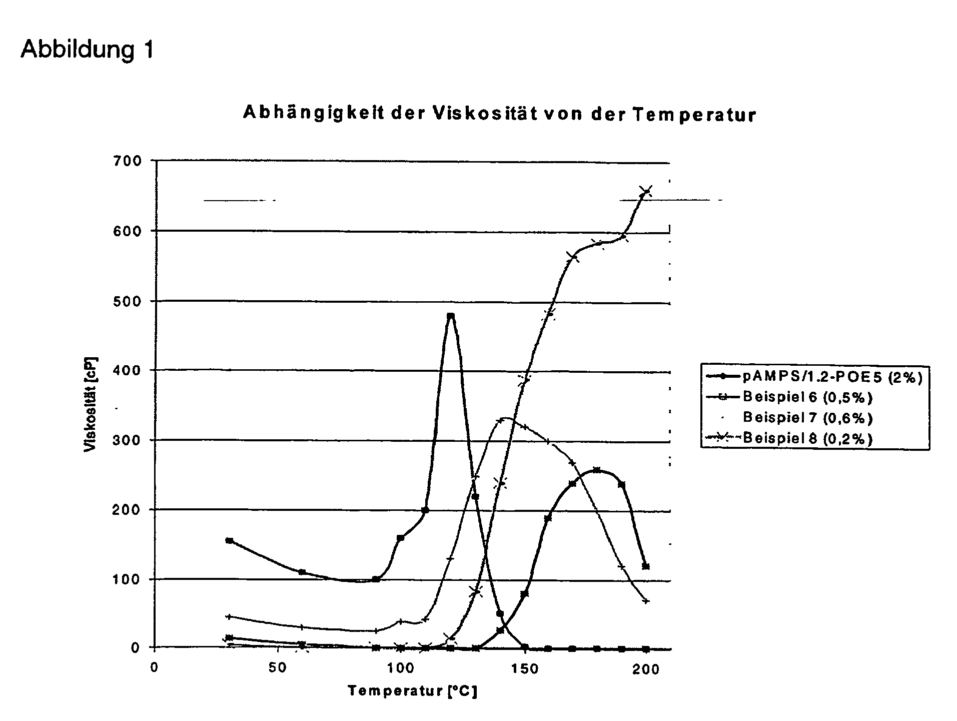

Weiterhin erkennt man deutlich, dass die erfindungsgemäßen Polymere aufgrund der hydrophoben Wechselwirkung der Alkylreste der Seitenketten bereits bei Raumtemperatur eine Grundviskosität zeigen. pAMPS/1.2-POE5 bildet eine solche Grundviskosität nicht aus. Des weiteren zeichnen sich die erfindungsgemäßen Polymere dadurch aus, dass die Breite des thermoassoziierenden Effekts steuerbar ist.

In den beiden folgenden Beispielen wird der Einfluss von Mischungen verschiedener Seitenarme in einem Polymer (Beispiel 18) bzw. der Einfluss von Mischungen verschiedener Polymere (Beispiel 19) dargestellt.

### Beispiel 18:

Diese Beispiel zeigt die Entwicklung der Viskosität bei unterschiedlichen Temperaturen für die Polymere aus Beispiel 9. Es kommen verschiedene Seitenarme in einem Polymer zum Einsatz (Abbildung 2).

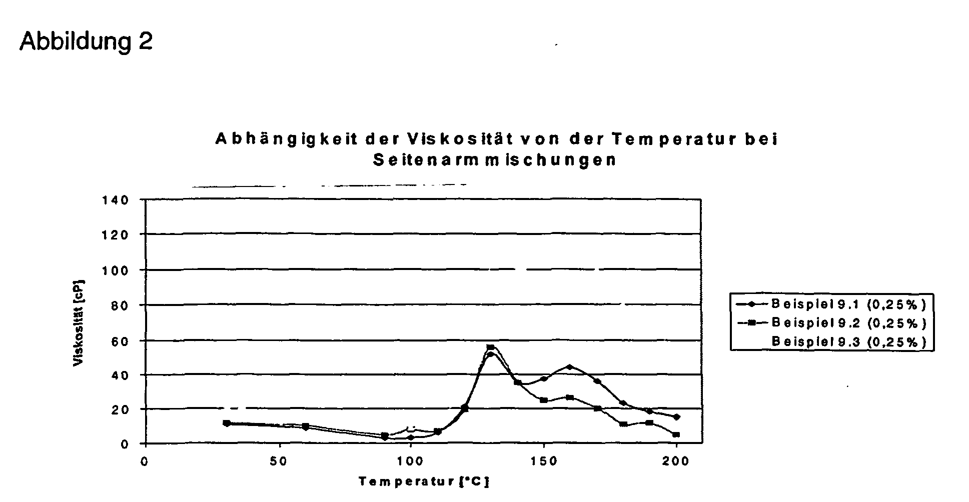

### Beispiel 19:

In Abbildung 3 ist gezeigt, wie sich das Viskositätsverhalten in Abhängigkeit von der Temperatur verändert. Die Beispiele 10.1 bis 10.3 sind unterschiedliche Mischungsverhältnisse der Polymere aus Beispiel 6 und Beispiel 7. Die Breite und Stärke des thermoassoziierenden Effekts lässt sich über das Mischungsverhältnis einstellen.

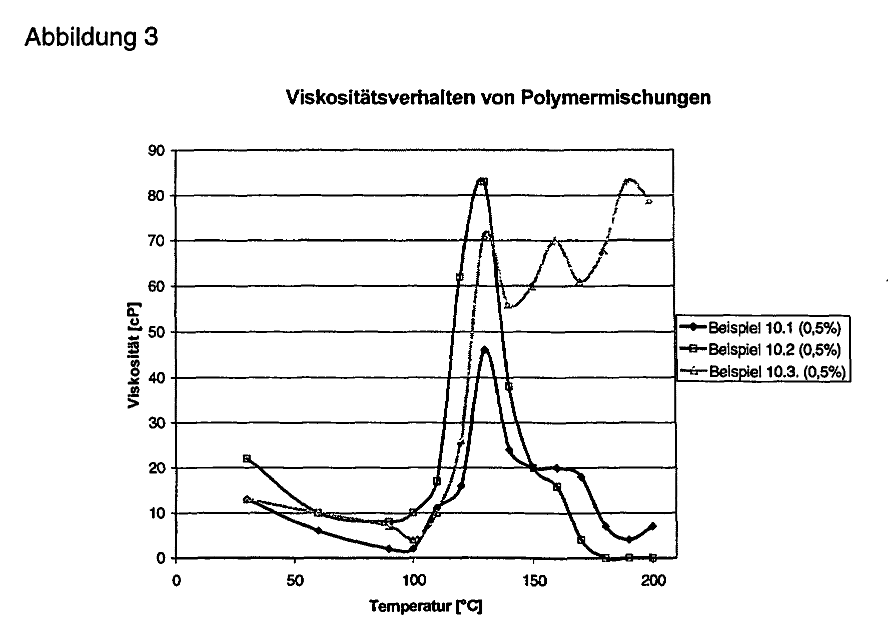

### Beispiele für Ölemulsionen

Die Testemulsionen werden bei 40°C, 45°C und 50°C über einen Zeitraum von 90 Tagen gelagert 3 verschiedenen Ölmischungen werden jeweils verwendet. Die Ölmischungen setzen sich folgendermaßen zusammen:

| Mischung I: | | Mischung II: | |
|---|---|---|---|
| Paraffinöl n.v. | 50 % | Cetiol V | 25 % |
| Isopropylpalmitat | 30 % | Squalan | 25 % |
| Sojaöl | 20 % | Myritol 318 Isopropylpalmitat | 25 % 25 % |

| Mischung III: | |
|---|---|
| Sojaöl | 50 % |
| Mandelöl | 20 % |
| Avocadoöl | 20 % |
| Jojobaöl | 1 0 % |

### Beurteilungsschlüssel:

0 = keine Emulgatorwirkung
1 = in 2 oder 3 Phasen getrennt
3 = Aufrahmung + Öl oder Wasser + Aufrahmung, dazwischen homogene Emulsion
4 = Wasserabscheidung, keine Aufrahmung, keine Ölabscheidung
5 = Ölabscheidung, keine Aufrahmung, keine Wasserabscheidung
6 = Aufrahmung
7 = schwach erkennbare Aufrahmung
8 = praktisch in Ordnung
9 = völlig homogene Emulsion

### Emulgierindex:

4.1 - 5.0 = sehr gut
3.1 - 4.0 = gut
2.1 - 3.0 = mittel
1.1 - 2.0 = schlecht
0 - 1.0 = sehr schlecht

### Testemulsionen mit unvernetzten erfindungsgemässen Polymeren

| Zusammensetzung | 20 | 21 | 22 | 23 | 24 | 25 | 26 | 27 | 28 | 29 | 30 | 31 |
|---|---|---|---|---|---|---|---|---|---|---|---|---|
| Polymer It. Bsp. 2 | - | - | - | 1,0 | - | - | 1,0 | - | - | 0,5 | - | - |
| Polymer It. Bsp. 1 | - | 1,0 | 1,0 | - | - | 1,0 | - | - | 1,0 | - | - | 0,5 |
| Polymer It. Bsp. 6 | 1,0 | - | - | - | 1,0 | - | - | 1,0 | - | - | 0,5 | - |
| Ölmischung I | 19,0 | 19,0 | - | - | - | - | - | - | - | - | - | - |
| Ölmischung II | - | - | 19,0 | - | - | - | - | - | - | - | - | - |
| Ölmischung III | - | - | - | 19,0 | 19,0 | 19,0 | 27,0 | 27,0 | 27,0 | 27,0 | 27,0 | 27,0 |
| Carbopol 980 | 0,3 | 0,3 | 0,3 | 0,3 | 0,3 | 0,3 | - | - | - | - | - | - |
| Natronlauge 10%ig | 1,2 | 1,2 | 1,2 | 1,2 | 1,2 | 1,2 | - | - | - | - | - | - |
| Wasser | Ad 100 | Ad 100 | Ad 100 | Ad 100 | Ad 100 | Ad 100 | Ad 100 | Ad 100 | Ad 100 | Ad 100 | Ad 100 | Ad 100 |
| Emulgierindex | 3,9 | 4,0 | 4,0 | 3,9 | 3,8 | 3,7 | 2,8 | 2,5 | 2,8 | 2,8 | 2,6 | 2,7 |
| Emulgierwirkung | gut | gut | gut | gut | gut | gut | mäßig | mäßig | mäßig | mäßig | mäßig | mäßig |

Der Stabilitätstest zeigt, dass die unvernetzten erfindungsgemäßen Polymere in Kombination mit zusätzlichem Konsistenzgeber stabile Emulsionen liefern.

### Testemulsionen mit vernetzten erfindungsgemäßen Polymeren

| Zusammensetzung | 32 | 33 | 34 | 35 | 36 | 37 | 38 | 39 | 40 |
|---|---|---|---|---|---|---|---|---|---|
| Polymer It. Bsp. 3 | 1 | 1 | 1 | 1 | 1 | 1 | 0,75 | 0,75 | 0,75 |
| Ölmischung I | 27 | | | 27 | | | 27 | | |
| Ölmischung II | | 27 | | | 27 | | | 27 | |
| Ölmischung III | | | 27 | | | 27 | | | 27 |
| Carbopol 980 | 0,3 | 0,3 | 0,3 | - | - | - | - | - | - |
| Natronlauge 10%ig | 1,2 | 1,2 | 1,2 | - | - | - | - | - | - |
| Wasser | Ad 100 | Ad 100 | Ad 100 | Ad 100 | Ad 100 | Ad 100 | Ad 100 | Ad 100 | Ad 100 |
| Emulgierindex | 4,7 | 4,8 | 4,7 | 4,6 | 4,4 | 4,5 | 4,5 | 4,6 | 4,6 |
| Emulgierwirkung | Sehr gut | Sehr gut | Sehr gut | Sehr gut | Sehr gut | Sehr gut | Sehr gut | Sehr gut | Sehr gut |

Der Stabilitätstest zeigt, dass die vernetzten erfindungsgemäßen Polymere gleichzeitig als Konsistenzgeber und als Emulgator mit sehr guter Emulgiereigenschaften wirken. Ohne zusätzlichen Co-Emulgator und ohne zusätzliche Konsistenzgeber werden stabile Emulsionen erhalten.

### Formulierungsbeispiele, alle Angaben in Gew.-%

### Beispiel 41: O/W - Hautmilch

### Zusammensetzung:

| | | |
|---|---|---|
| A | ^{®}Polymer It. Bsp. 2 | 0.50 % |
| | Mineralöl, niedrig viskos | 5.00 % |
| | ^{®}Miglyol 812 (Dynamit Nobel) | 4.00 % |
| | Capryl/Caprin Triglyceride | |
| | Isopropylpalmitat | 6.00 % |
| | Soyaöl | 3.00 % |
| | Jojobaöl | 2.00 % |
| B | ^{®}Aristoflex AVC (Clariant) | 30 % |
| | AMPS / VIFA - Copolymer | |
| C | ^{®}Hostapon KCG (Clariant) | 1.00 % |
| | Natriumcocoylglutamat | |
| | Wasser | ad 100 % |
| | Glycerin | 3.00 % |
| | Natriumhydroxid (10 % in water) | 1.20 % |
| D | Duftstoffe | 0.30 % |

### Herstellung

I B in A einrühren, C hinzugeben und gut verrühren.
II D in I einrühren
III Emulsion homogenisieren

### Beispiel 42: O/W - Hautmilch

### Zusammensetzung

| | | |
|---|---|---|
| A | Polymer It. Bsp. 1 | 0.50 % |
| | Isopropylpalmitat | 4,00 % |
| | Mandelöl 5,00 % | 4.00 % |
| | Weizenkeimöl | 1.00 % |
| | ^{®}Cetion SN (Henkel) | 8,00 % |
| | Cetearylisononanoat | |
| | | |
| B | ^{®}Aristoflex AVC (Clariant) | 0.30 % |
| | AMPS/VIFA-Copolymer | |
| C | Wasser | ad 100 % |
| D | Duftstoffe | 0.30 % |

### Herstellung

I A und B mischen, dann C hinzugeben
II D zu I hinzurühren
III Emulsion homogenisieren

### Beispiel 43: O/W After-Sun-Milch

### Zusammensetzung:

| | | |
|---|---|---|
| A | Polymer It. Bsp. 1 | 1,00% |
| | Isopropylpalmitat | 5,00 % |
| | ^{®}Cetion SN (Henkel) | 4,00 % |
| | Cetearylisononanoat | |
| | Sojaöl | 4,00 % |
| | ^{®}Miglyol 812 (Dynamit Nobel) | 3,00 % |
| | Capryl/Caprin Triglyceride | |
| | Jojobaöl | 3,00 % |
| | Weizenkeimöl | 1,00 % |
| D | ^{®}AQUAMOLLIN BC Plv. hochkonz. (Clariant) | 0,10 % |
| | Ethylendiamine Tetraacetic Acid Sodium Salt | |
| | Citronensäure (10 %ig) | 0,30 % |
| | Wasser | 68,80 % |
| | Glycerin | 3,00 % |
| | ALLANTOIN (Clariant) | 0,20 % |
| | Konservierungsmittel | q.s. |
| E | Ethanol | 1 ,50 % |
| | Parfümöl | 0,30 % |

### Herstellung

I Die Komponenten von A homogen verrühren
II Bei ca. 35°C D in I einrühren.
III Die Emulsion homogenisieren.

### Beispiele für Wasser in Öl Formulierungen

### Beispiel 44: Reaktion nach allgemeiner Polymerisationsvorschrift

| Reaktand | Menge (g) |
|---|---|
| Genapol^{®}T-250-methacrylat | 90 |
| NH₃-neutralisiertes AMPS | 10 |
| TMPTA | 1,5 |
| t-Butanol | 300 |
| Dilauroylperoxid (Initiator) | 1 |

### Beispiel 45: W/O Creme

### Zusammensetzung

| | | |
|---|---|---|
| A | ^{®}HOSTACERIN DGI (Clariant) | 4.00 % |
| | Polyglyceryl-2 Sesquiisostearate | |
| | Beeswax | 2.00 % |
| | Polymer It. Bsp 18 | 1,5 % |
| | Mineral Oil, low viscosity | 5.00 % |
| | Vaseline | 10.00 % |
| | ^{®}Cetiol V (Henkel KGaA) | 5.00 % |
| | Decyl Oleate | |
| B | 1,2-Propylene glycol | 3.00 % |
| | Wasser | 69,10 % |
| | Preservative | q.s. |
| C | Fragrance | 0.40 % |

### Herstellung

I A bei 80°C aufschmelzen
II B auf 80°C erhitzen.
III II in I einrühren.
IV Stir until cool.
V At 35°C add C to IV.

### Beispiele für Tensid Formulierungen

### Beispiel 46: body wash

### Zusammensetzung

| | | |
|---|---|---|
| A | ^{®}GENAPOL LRO liquid (Clariant) | 40.00 % |
| | Sodium Laureth sulfate | |
| B | Fragrance | 0.30 % |
| C | Wasser | 52.70 % |
| | Dyestuff | q.s. |
| | Preservative | q.s. |
| | ^{®}GENAGEN LDA (Clariant) | 6.00 % |
| | Disodium Lauroamphodiacetate | |
| | Citric Acid | q.s. |
| D | Polymer It. Bsp. 6 | 1.00 % |

### Herstellung

I B in A einrühren
II Komponenten aus C nacheinander zu I zugeben
III pH auf 5.5 einstellen
IV Einstellen der Viskosität durch Einrühren von D in II

### Beispiel 47: Baby Shampoo

### Zusammensetzung

| | | |
|---|---|---|
| A | Wasser | 60.70 % |
| | ^{®}GENAPOL ZRO liquid (Clariant) | 25.00 % |
| | Sodium Laureth Sulfate | |
| | ^{®}HOSTAPON CLG (Clariant) | 8.00 % |
| | Sodium Lauroyl Glutamate | |
| | ^{®}GENAPOL SBE (Clariant) | 5.00 % |
| | Disodium Laureth Sulfosuccinate | |
| | Fragrance | 0.30 % |
| | Dyestuff solution | q.s. |
| | Preservative | q.s. |
| B | Polymer It. Beispiel 4 | 1,00 % |

### Herstellung

I B in A lösen
II gegebenenfalls pH einstellen

### Beispiel 48: Antischuppen Shampoo, klar

### Zusammensetzung

| | | |
|---|---|---|
| A | ^{®}OCTOPIROX (Clariant) | 0.50 % |
| | Piroctone Olamine | |
| B | Wasser | 10.00 % |
| C | ^{®}GENAPOL LRO LIQUID (Clariant) | 30.00 % |
| | Sodium Laureth Sulfate | |
| D | ^{®}Belsil DMC 6032 (Wacker Chemie) | 1.50 % |
| | Dimethicone Copolyol Acetate | |
| | Fragrance | 0.30 % |
| E | ^{®}ALLANTOIN (Clariant) | 0.30 % |
| F | Wasser | 46.40 % |
| G | Dyestuff solution | q.s. |
| | Panthenol (Hoffmann La Roche) | 1.00 % |
| | ^{®}GENAGEN CAB (Clariant) | 8.00 % |
| | Cocamidopropyl Betaine | |
| H | Polymer It. Beispiel 7 | 1.10 % |

### Herstellung

I A mit B mischen
II C in I einrühren bis klare Lösung
III Komponenten aus D nacheinander in I geben
IV E in F unter Erwärmen einrühren und dann in I einrühren
V Komponenten aus G nacheinander in I geben
VI gegebenenfalls pH einstellen
VII Einstellen der Viskosität durch Einrühren von H in I

### Beispiel 49: Antischuppen Shampoo, perlglänzend

### Zusammensetzung

| | | |
|---|---|---|
| A | Wasser | 38.7 % |
| B | ^{®}HOSTAPON SCI-65 (Clariant) | 3.00 % |
| | Sodium Cocoyl Isethionate | |
| C | ^{®}GENAPOL LRO liquid (Clariant) | 35.00 % |
| | Sodium Laureth Sulfate | |
| | ^{®}HOSTAPON KCG (Clariant) | 5.00 % |
| | Sodium Cocoyl Glutamate | |
| | ^{®}Belsil DMC 6032 (Wacker) | 1.00 % |
| | Dimethicone Copolyol Acetate | |
| | Fragrance | 0.30 % |
| | ^{®}GENAGEN CAB (Clariant) | 9.00 % |
| | ^{®}Cocamidopropyl Betaine | |
| | GENAPOL TSM (Clariant) | 4.00 % |
| | PEG-3 Distearate (and) Sodium Laureth Sulfate | |
| | Merquat 550 | 0.50 % |
| | Polyquatemium-7 | |
| | Zinc Omadine FPS (Olin) | |
| | Zinc Pyrithione (48%ig) | 2.50 % |
| | Polymer nach Bsp. 9.1 | 1.00 % |
| | Dyestuff solution | q.s. |
| | Preservative | q.s. |

### Herstellung

I Bei 80°C B in A lösen
II Nach Abkühlen auf ca. 35°C, Komponenten C nacheinander zugeben

### Beispiele für Gele

### Beispiel 50: Haargel mit konditionierenden Eigenschaften

### Zusammensetzung

| | | |
|---|---|---|
| A | Wasser | 92.00 % |
| | Panthenol | 1.50 % |
| | UVAsorb S5 | 0.05 % |
| | Benzophenone-4 | |
| | Dyestuff solution | q.s. |
| | Preservative | q.s. |
| B | ^{®}Emulsogen HCO 040 (Clariant) | 0.50 % |
| | PEG-40 Hydrogenated Castor Oil | |
| | parfume | q.s. |
| C | Polymer nach Beispiel 3 | 2.00 % |
| D | Gafquat 755N (ISP) | 2.50 % |
| | Polyquaternium-11 | |

### Herstellung

I Komponenten A mischen
II Komponenten B mischen und zu I geben
III C zu D zu I geben

### Beispiel 51: Haargel mit starkem Halt

### Zusammensetzung

| | | |
|---|---|---|
| A | Wasser | 91.50 % |
| | PVP K-30 (ISP) | 4.00 % |
| | PVP | |
| | Panthenol | 0.50 % |
| | UVAsorb S5 | 0.05 % |
| | Benzophenone-4 | |
| | Dyestuff solution | q.s. |
| | Preservative | q.s. |
| B | Abil B 8851 (Goldschmidt) | 1.00 % |
| | Dimethicone Copolyol | |
| | ^{®}Emulsogen HCO 040 (Clariant) | 0.50 % |
| | PEG-40 Hydrogenated Castor Oil | |
| | parfume | q.s. |
| C | Polymer nach Beispiel 3 | 2.50 % |

### Herstellung

I Komponenten A mischen
II Komponenten B in I geben
III Komponenten C in I geben

## Patentansprüche

1. Wasserlösliche Polymere, herstellbar durch radikalische Copolymerisation von
A) einem oder mehreren Makromonomeren der Formel (1 )
R¹-Y-(R²-O)ₓ -R³ (1)
worin R¹ einen Acryl- oder Methacrylrest; R² (C₂-C₄)-Alkylen; x eine ganze Zahl zwischen 1 und 500; Y = O, S, PH oder NH; und R³ einen gesättigten oder ungesättigten linearen oder verzweigten aliphatischen oder cycloaliphatischen (C₆-C₃₀)-Kohlenwasserstoffrest bedeuten und
B) einem oder mehreren olefinisch ungesättigten Comonomeren, die Sauerstoff, Stickstoff, Schwefel, Phosphor, Chlor und/oder Fluor enthalten.

2. Polymere nach Anspruch 1, **dadurch gekennzeichnet, dass** es sich bei dem Rest R³ in Formel (1) um einen aliphatischen oder cycloaliphatischen (C₆-C₂₂)-Kohlenwasserstoffrest, der gesättigt oder ungesättigt sein kann, handelt.

3. Polymere nach Anspruch 1 oder 2, **dadurch gekennzeichnet, dass** es sich bei dem Rest R³ in Formel (1) um einen aliphatischen oder cycloaliphatischen (C₁₂-C₂₂)-Kohlenwasserstoffrest, der gesättigt oder ungesättigt sein kann, handelt.

4. Polymere nach mindestens einem der Ansprüche 1 bis 3, **dadurch gekennzeichnet, dass** es sich bei den Comonomeren B) um olefinisch ungesättigte Säuren und/oder deren Salze mit ein- und zweiwertigen Gegenionen, besonders bevorzugt Acrylamidopropylmethylensulfonsäure (AMPS); Ester der Acryl- und der (Meth)acrylsäure mit aliphatischen, aromatischen oder cycloaliphatischen Alkoholen mit einer Kohlenstoffzahl von 1 bis 22; Ester der Acryl- und der (Meth)acrylsäure mit Alkylethoxylaten, offenkettige und cyclische N-Vinylamide mit einer Ringgröße von 4 bis 9 Atomen; Amide der Acryl- und der Methacrylsäure; 2-Vinylpyridin; 4-Vinylpyridin; Vinylacetat; Methacrylsäureglycidylester; Acrylnitril; Vinylchlorid; Vinylidenchlorid; Tetrafluorethylen und/oder DADMAC handelt.

5. Polymere nach mindestens einem der Ansprüche 1 bis 4, herstellbar durch radikalische Copolymerisation von
A) einem oder mehreren Makromonomeren, ausgewählt aus der Gruppe der Ester der (Meth)acrylsäure mit Alkylethoxylaten, die 5 bis 80 EO-Einheiten und (C₁₀-C₂₂)-Alkylreste umfassen, und
B) einem oder mehreren olefinisch ungesättigten Comonomeren, ausgewählt aus der Gruppe Acrylamidopropylmethylensulfonsäure (AMPS), Natrium- und Ammoniumsalze der Acrylamidopropylmethylensulfonsäure (AMPS), Acrylamid, N-Vinylformamid, N-Vinylmethylacetamid und Natriummethallylsulfonat.

6. Polymere nach mindestens einem der Ansprüche 1 bis 5, **dadurch gekennzeichnet, dass** der Anteil an Makromonomeren 50,1-99,9 mol-% beträgt.

7. Polymere nach mindestens einem der Ansprüche 1 bis 5, **dadurch gekennzeichnet, dass** der Anteil an Makromonomeren 0,1-50 mol-% beträgt.

8. Polymere nach mindestens einem der Ansprüche 1 bis 7, **dadurch gekennzeichnet, dass** sie durch Copolymerisation von mindestens einem Vernetzer mit mindestens zwei ungesättigten Doppelbindungen vernetzt sind.

9. Polymere nach mindestens einem der Ansprüche 1 bis 8, **dadurch gekennzeichnet, dass** sie aus einem wasserlöslichen Polymerskelett und Makromonomeren A) mit wärmeempfindlichen Seitenketten, die in Wasser ein LCST-Verhalten aufweisen, bestehen, und deren wässrige Lösung eine Viskosität hat, die oberhalb einer bestimmten Schwellentemperatur mit steigender Temperatur zunimmt oder ungefähr konstant bleibt.

10. Polymere nach mindestens einem der Ansprüche 1 bis 8, **dadurch gekennzeichnet, dass** ihre 1 %igen wässrigen Lösungen bei Raumtemperatur eine Viskosität von 20 000 mPas bis 100 000 mPas zeigen und die Lösungen kein thermoassozierendes Verhalten zeigen.

11. Polymere nach mindestens einem der Ansprüche 1 bis 10, **dadurch gekennzeichnet, dass** sie durch Fällungspolymerisation in tert.-Butanol hergestellt werden.

12. Wässrige Zubereitungen, wässrig-alkoholische Zubereitungen, wässrig-tensidische Zubereitungen, Emulsionen und Suspensionen, enthaltend Polymere nach mindestens einem der Ansprüche 1 bis 11.

13. Zubereitungen, Emulsionen und Suspensionen nach Anspruch 12, **dadurch gekennzeichnet, dass** es sich dabei um kosmetische und pharmazeutische Mittel handelt.

14. Zubereitungen, Emulsionen und Suspensionen nach Anspruch 12 oder 13, **dadurch gekennzeichnet, dass** sie, bezogen auf die fertige Formulierung, 0,05 bis 10 Gew.-% an Polymeren enthalten.

15. Verwendung von Polymeren nach mindestens einem der Ansprüche 1 bis 11 als Verdicker, Dispergiermittel, Suspendiermittel, Emulgator, Stabilisator und/oder Konsistenzgeber.

16. Verwendung von Polymeren nach mindestens einem der Ansprüche 1 bis 11 als Adjuvants in Pflanzenschutzformulierungen.

## Claims

1. A water-soluble polymer preparable by free-radical copolymerization of
A) one or more macromonomers of the formula (1)
R¹-Y-(R²-O)ₓ-R³ (1)
in which R¹ is an acrylic or methacrylic radical; R² is (C₂-C₄)-alkylene; x is an integer between 1 and 500; Y = O, S, PH or NH; and R³ is a saturated or unsaturated linear or branched aliphatic or cycloaliphatic (C₆-C₃₀)-hydrocarbon radical, and
B) one or more olefinically unsaturated comonomers which contain oxygen, nitrogen, sulfur, phosphorus, chlorine and/or fluorine.

2. The polymer as claimed in claim 1, wherein the radical R³ in formula (1) is an aliphatic or cycloaliphatic (C₆-C₂₂)-hydrocarbon radical which may be saturated or unsaturated.

3. The polymer as claimed in claim 1 or 2, wherein the radical R³ in formula (1) is an aliphatic or cycloaliphatic (C₁₂-C₂₂)-hydrocarbon radical which may be saturated or unsaturated.

4. The polymer as claimed in at least one of claims 1 to 3, wherein the comonomers B) are olefinically unsaturated acids and/or salts thereof containing mono- and divalent counterions, particularly preferably acrylamidopropylmethylenesulfonic acid (AMPS); esters of acrylic and (meth)acrylic acid with aliphatic, aromatic or cycloaliphatic alcohols having a carbon number from 1 to 22; esters of acrylic and (meth)acrylic acid with alkyl ethoxylates, open-chain and cyclic N-vinylamides having a ring size of from 4 to 9 atoms; amides of acrylic acid and of methacrylic acid; 2-vinylpyridine; 4-vinylpyridine; vinyl acetate; glycidyl methacrylate; acrylonitrile; vinyl chloride; vinylidene chloride; tetrafluoroethylene and/or DADMAC.

5. The polymer as claimed in at least one of claims 1 to 4, preparable by free-radical copolymerization of
A) one or more macromonomers chosen from the group of esters of (meth)acrylic acid with alkyl ethoxylates which include 5 to 80 EO units and (C₁₀-C₂₂)-alkyl radicals, and
B) one or more olefinically unsaturated comonomers chosen from the group consisting of acrylamidopropylmethylenesulfonic acid (AMPS), sodium and ammonium salts of acrylamidopropylmethylenesulfonic acid (AMPS), acrylamide, N-vinylformamide, N-vinylmethylacetamide and sodium methallylsulfonate.

6. The polymer as claimed in at least one of claims 1 to 5, wherein the proportion of macromonomers is 50.1-99.9 mol%.

7. The polymer as claimed in at least one of claims 1 to 5, wherein the proportion of macromonomers is 0.1-50 mol%.

8. The polymer as claimed in at least one of claims 1 to 7, which has been crosslinked by copolymerization of at least one crosslinker having at least two unsaturated double bonds.

9. The polymer as claimed in at least one of claims 1 to 8, which consists of a water-soluble polymer skeleton and macromonomers A) having heat-sensitive side chains, which have LCST behavior in water, and the aqueous solution of which has a viscosity which, above a certain threshold temperature, increases or remains roughly constant with increasing;temperature.

10. The polymer as claimed in at least one of claims 1 to 8, wherein its 1% strength aqueous solution at room temperature exhibits a viscosity of from 20,000 mPas to 100,000 mPas, and the solution does not exhibit thermoassociative behavior.

11. The polymer as claimed in at least one of claims 1 to 10, which is prepared by precipitation polymerization in tert-butanol.

12. An aqueous preparation, aqueous-alcoholic preparation, aqueous/surface-active preparation, emulsion or suspension comprising polymers as claimed in at least one of claims 1 to 11.

13. The preparation, emulsion or suspension as claimed in claim 12, which is a cosmetic or pharmaceutical composition.

14. The preparation, emulsion or suspension as claimed in claim 12 or 13, which, based on the finished formulation, comprises 0.05 to 10% by weight of polymers.

15. The use of polymers as claimed in at least one of claims 1 to 11 as thickeners, dispersing agents, suspending agents, emulsifiers, stabilizers and/or bodying agents.

16. The use of polymers as claimed in at least one of claims 1 to 11 as adjuvants in crop protection formulations.

## Revendications

1. Polymères hydrosolubles, pouvant être obtenus par copolymérisation radicalaire
A) d'un ou plusieurs macromonomères de formule (1)
R¹-Y-(R²-O)ₓ-R³ (1)
dans laquelle R¹ représente un radical acryloyle ou méthacryloyle ; R² représente un groupe alkylène en C₂-C₄ ; x représente un nombre entier compris entre 1 et 500 ; Y = O, S, PH ou NH ; et R³ représente un radical hydrocarboné aliphatique linéaire ou ramifié ou cycloaliphatique en C₆-C₃₀, saturé ou insaturé, et
B) d'un ou plusieurs comonomères à insaturation oléfinique, qui contiennent de l'oxygène, de l'azote, du soufre, du phosphore, du chlore et/ou du fluor.

2. Polymères selon la revendication 1, **caractérisés en ce que** le radical R³ dans la formule (1) consiste en un radical hydrocarboné aliphatique ou cycloaliphatique en C₆-C₂₂, qui peut être saturé ou insaturé.

3. Polymères selon la revendication 1 ou 2, **caractérisés en ce que** le radical R³ dans la formule (1) consiste en un radical hydrocarboné aliphatique ou cycloaliphatique en C₁₂-C₂₂, qui peut être saturé ou insaturé.

4. Polymères selon au moins l'une des revendications 1 à 3, **caractérisés en ce que** les comonomères B) consistent en des acides à insaturation oléfinique et/ou en leurs sels avec des ions opposés mono- ou divalents, de façon particulièrement préférée en l'acide acrylamidopropylméthylènesulfonique (AMPS) ; en des esters de l'acide acrylique et de l'acide (méth)acrylique avec des alcools aliphatiques, aromatiques ou cycloaliphatiques ayant un nombre d'atomes de carbone de 1 à 22 ; en des esters de l'acide acrylique et de l'acide (méth) acrylique avec des alkyléthoxylates, des N-vinylamides à chaîne ouverte ou cycliques ayant une taille de cycle de 4 à 9 atomes ; en des amides de l'acide acrylique et de l'acide méthacrylique ; en la 2-vinylpyridine ; la 4-vinylpyridine ; l'acétate de vinyle ; le méthacrylate de glycidyle ; l'acrylonitrile ; le chlorure de vinyle ; le chlorure de vinylidène ; le tétrafluoroéthylène et/ou le DADMAC.

5. Polymères selon au moins l'une des revendications 1 à 4, pouvant être préparés par copolymérisation radicalaire
A) d'un ou plusieurs macromonomères, choisis dans le groupe des esters de l'acide (méth)acrylique avec des alkyléthoxylates qui comprennent de 5 à 80 groupes OE et des radicaux alkyle en C₁₀-C₂₂, et
B) d'un ou plusieurs comonomères à insaturation oléfinique, choisis dans l'ensemble constitué par l'acide acrylamidopropylméthylènesulfonique (AMPS), les sels de sodium et d'ammonium de l'acide acrylamidopropylméthylènesulfonique (AMPS), l'acrylamide, le N-vinylformamide, le N-vinylméthyl-acétamide et le méthallylsulfonate de sodium.

6. Polymères selon au moins l'une des revendications 1 à 5, **caractérisés en ce que** la teneur en macromonomères vaut de 50,1 à 99,9 % en moles.

7. Polymères selon au moins l'une des revendications 1 à 5, **caractérisés en ce que** la teneur en macromonomères vaut de 0,1 à 50 % en moles.

8. Polymères selon au moins l'une des revendications 1 à 7, **caractérisés en ce qu'**ils sont réticulés par copolymérisation d'au moins un agent de réticulation comportant au moins deux doubles liaisons insaturées.

9. Polymères selon au moins l'une des revendications 1 à 8, **caractérisés en ce qu'**ils sont constitués d'un squelette polymère hydrosoluble et de macromonomères A) à chaînes latérales sensibles à la chaleur, qui présentent dans l'eau un comportement LCST, et dont la solution aqueuse a une viscosité qui, au-dessus d'une certaine température seuil, avec une température croissante augmente ou reste approximativement constante.

10. Polymères selon au moins l'une des revendications 1 à 8, **caractérisés en ce que** leurs solutions aqueuses à 1 % présentent à la température ambiante une viscosité de 20 000 mPa.s à 100 000 mPa.s et les solutions ne présentent pas de comportement lié à la chaleur.

11. Polymères selon au moins l'une des revendications 1 à 10, **caractérisés en ce qu'**ils sont préparés par polymérisation par précipitation dans du tert-butanol.

12. Préparations aqueuses, préparations aqueuses-alcooliques, préparations aqueuses contenant des tensioactifs, émulsions et suspensions, contenant des polymères selon au moins l'une des revendications 1 à 11.

13. Préparations, émulsions et suspensions selon la revendication 12, **caractérisées en ce qu'**il s'agit de compositions cosmétiques et pharmaceutiques.

14. Préparations, émulsions et suspensions selon la revendication 12 ou 13, **caractérisées en ce qu'**elles contiennent, par rapport à la composition finale, de 0,05 à 10 % en poids de polymères.

15. Utilisation de polymères selon au moins l'une des revendications 1 à 11, en tant qu'épaississant, dispersant, agent de mise en suspension, émulsifiant, stabilisant et/ou agent de consistance.

16. Utilisation de polymères selon au moins l'une des revendications 1 à 11, en tant qu'adjuvants dans des compositions phytosanitaires.
